# FASCICULE DE BREVET EUROPEEN

(11) **EP 3 995 131 B1**
(45) Date de publication et mention de la délivrance du brevet: **26.06.2024**
(21) Numéro de dépôt: 21204830.0
(22) Date de dépôt: 26.10.2021
(51) Int. Cl.: A61K 9/00, A61K 9/107, A61K 47/36, A61K 8/60, A61Q 19/00

(54) **COMPOSITION POUR LE TRAITEMENT DE LA ROSACEE ET/OU DE LA TELANGIECTASIE**
ZUSAMMENSETZUNG ZUR BEHANDLUNG VON ROSACEA UND/ODER TELANGIEKTASIE
COMPOSITION FOR THE TREATMENT OF ROSACEA AND/OR TELANGIECTASIA

(30) Priorité: 10.11.2020 FR 2011519
(43) Date de publication de la demande: 11.05.2022
(73) Titulaire: NAOS Institute of Life Science, 13290 Aix-en-Provence (FR); Thorel, Jean-Noël, 75014 Paris (FR)
(72) Inventeur: THOREL, Jean-Noël, 75014 PARIS (FR)
(74) Mandataire: Cabinet Laurent & Charras

(56) Documents cités:
- CN-A- 103 735 458
- FR-A1- 3 022 139
- US-A- 5 912 330
- Anonymous: "MALTOOLIGOSYL GLUCOSIDE", , 17 mai 2017 (2017-05-17), XP055817720, Extrait de l'Internet: URL:https://web.archive.org/web/2017051722 2147/https://cosmetics.specialchem.com/inc i/maltooligosyl-glucoside [extrait le 2021-06-24]
- LI YAN-FEI ET AL: "Inhibitory effect of timolol on topical glucocorticoid-induced skin telangiectasia", MOLECULAR MEDICINE REPORTS , vol. 18 1 janvier 2018 (2018-01-01), pages 2823-2831, XP055818411, GR ISSN: 1791-2997, DOI: 10.3892/mmr.2018.9266 Extrait de l'Internet: URL:https://www.ncbi.nlm.nih.gov/pmc/artic les/PMC6102693/pdf/mmr-18-03-2823.pdf

## Description

### Domaine technique de l'invention

L'invention se rapporte au domaine des soins dermatologiques, et plus particulièrement du traitement des affections inflammatoires cutanées. Plus précisément, l'invention concerne une composition, avantageusement cosmétique, pour le traitement de la rosacée et/ou de la télangiectasie

### Arrière-plan technologique

La peau constitue la principale barrière qui sépare l'organisme du milieu extérieur et se compose de la superposition de trois couches principales, respectivement de la plus profonde à la plus superficielle : l'hypoderme, le derme et l'épiderme. Plusieurs facteurs ont une incidence sur la santé de la peau. Ainsi et en raison de son interaction constante avec l'environnement, la peau subit de multiples agressions (virus, bactéries, UV, agressions chimiques, températures extrêmes...) qui altèrent son équilibre. En parallèle de ces atteintes exogènes, chaque individu présente des facteurs internes ayant une incidence sur la santé de la peau à savoir, le patrimoine génétique ou encore les hormones.

Ces différents facteurs exogènes et endogènes peuvent être à l'origine de pathologies dermatologiques, notamment des affections inflammatoires cutanées.

Ces affections inflammatoires cutanées, comme la rosacée, engendrent des sensations d'inconfort physique. De plus, elles peuvent devenir une entrave à la vie sociale. En d'autres termes, ces affections, notamment la rosacée et/ou la télangiectasie, sont à l'origine d'inconvénients cosmétiques et esthétiques.

Concernant la rosacée, toutes les formes sont visibles et les épisodes de poussée inflammatoire comme ceux de rémission sont imprévisibles, ce qui rend la maladie difficile à maîtriser. La rosacée a donc un véritable impact sur le moral, sur les relations sociales et la confiance en soi.

La rosacée et les pathologies cutanées liées à la rosacée figurent parmi la liste des maladies inflammatoires chroniques cutanées. La rosacée se caractérise par des rougeurs sur les joues, le nez, le front et le menton. La rosacée et les maladies de peau liées à la rosacée touchent les petits vaisseaux du visage. Elles ne se manifestent donc pas seulement par des rougeurs, mais également par des vaisseaux apparents, et dans certains cas plus sévères par des boutons et un épaississement de la peau.

La rosacée et les maladies associées à la rosacée sensibilisent généralement la peau et dans certains cas, la rendent intolérante.

En plus de la rosacée, il existe donc différentes formes de maladies de la peau liées à la rosacée parmi lesquelles la télangiectasie, les flushs, la couperose, la rosacée oculaire, la rosacée papulo-pustuleuse, la forme hypertrophique, la rosacée phymateuse ou encore la rosacée érythèmatotélangiectasique. Toutefois, la rosacée et les maladies associées à cette pathologie de la peau sont à différencier d'autres affections de la peau comme le psoriasis (plaques rouges qui desquament) ou la dermatite séborrhéique (qui touche les glandes sébacées).

La télangiectasie est une pathologie caractérisée par des veinules qui deviennent visibles sous la peau car elles sont dilatées. Ces petits vaisseaux apparaissent rouges ou violacés. Il peut également s'agir de symptômes associés à la rosacée vasculaire ou couperose.

La rosacée se manifeste par un érythème (rougeurs occasionnelles ou persistantes), une couperose (appelée également télangiectasie) et/ou des papulopustules (lésions cutanées semblable à des lésions d'acné mais sans comédon). A des stades plus ultimes, la rosacée peut s'accompagner d'une hypertrophie plutôt localisée sur le nez (rhinophyma) ou d'une atteinte oculaire.

La prévalence de la rosacée reste très variable entre les pays, les populations étudiées et l'âge. En Europe, la population touchée est estimée entre 2 et 10 %. A titre d'exemple, en Allemagne la prévalence est estimée à 12,3 %, en Angleterre à 1,8%, aux Etats-Unis à 1,34 %, et en France entre 2 et 3 %.

Les études épidémiologiques montrent que les personnes à peaux et yeux clairs sont majoritairement atteintes. Toutefois, les populations avec un phototype V et VI peuvent l'être également.

Les causes exactes de la rosacée sont encore en cours de définition mais il a été solidement établi que cette condition est d'origine multifactorielle. Si la contribution de composante vasculaire est évidente, dans les dernières années elle semble être passée au second plan, derrière le rôle de l'immunité innée qui a été mis en avant par de nombreuses études fondamentales et clinico-biologiques. Selon ce scénario, maintenant favorisé par la plupart des chercheurs dans le domaine, une stimulation anormale de l'immunité innée aboutit à l'augmentation de médiateurs inflammatoires et également à une perturbation de la fonction barrière de la peau.

Parmi les médiateurs de l'immunité innée qui ont été impliqués de façon convaincante dans l'étiologie de la rosacée, un rôle clé a été attribué au peptide antimicrobien cathélicidine. La cathélicidine humaine est un peptide antimicrobien qui joue un rôle important dans la défense de la peau face aux pathogènes. Ce médiateur est sécrété sous forme de pro-peptide de 18 kDa, appelé hCAP18, biologiquement inactif. Le clivage protéolytique du côté C terminal par des protéases permet la libération de la forme antimicrobienne active dénommée LL-37 mais aussi d'autres fragments peptidiques.

Une corrélation crédible existe entre des niveaux élevés de LL-37 dans l'épiderme et l'apparition de la rosacée. Dans la peau normale, la teneur en peptide LL-37 est faible, tandis que dans la peau de patients atteints de rosacée, le peptide LL-37 est présent à un niveau 10 fois plus élevé que chez les témoins au niveau de l'épiderme.

Le peptide LL-37 cible les bactéries Gram + et Gram - et possède également une activité antifongique. En plus de son activité antimicrobienne, LL-37 présente des propriétés pro-inflammatoires via un effet chémoattractant sur les monocytes, les neutrophiles et les lymphocytes T et affecte l'angiogenèse. Cet effet pro-inflammatoire aide l'organisme à mieux répondre aux attaques de pathogènes, mais au même temps, peut exacerber l'irritation de la peau.

Le clivage de la protéine hCAP18 dans sa forme active pro-inflammatoire LL-37 est effectuée par la protéase kallicréine 5 (KLK-5). KLK-5 est une sérine protéase exprimée par les kératinocytes dans la peau normale. Il a été montré que la peau lésionnelle des sujets atteints de rosacée possède une expression du gène KLK-5 supérieure à celle de la peau des sujets contrôles sains. Il est intéressant de noter que cette surexpression est corrélée à celle de la cathélicidine.

Compte tenu de sa capacité à dégrader les protéines qui forment la composante extracellulaire des jonctions cellulaires dans le *stratum corneum,* l'enzyme KLK-5 joue également un rôle dans l'élimination des cornéocytes par desquamation. De par son rôle dans la desquamation, une activité protéolytique augmentée de KLK-5 entraîne alors une détérioration progressive de la fonction barrière.

L'activité enzymatique de KLK-5 est également régulée par les métalloprotéinases matricielles (MMPs). Plus précisément, l'activation de KLK-5 se produit après le clivage de sa forme de proenzyme par la MMP-9. Notamment, une expression accrue de MMP-2 et de MMP-9 a été observée dans la peau de patients atteints de rosacée. Une telle expression accrue de MMPs a des implications potentielles pour la dégénérescence par les UV observée dans la rosacée. De plus, l'initiation de l'activation de KLK-5 par les MMPs conduit à une activation supplémentaire de LL-37, la forme clivée de la cathélicidine, amplifiant de cette façon la cascade pro-inflammatoire.

Il a également été montré que l'enzyme KLK-5 est capable d'activer PAR-2 (récepteur activé par la protéase 2), Ce récepteur cellulaire est fortement exprimé par les kératinocytes de la couche granuleuse, où il est impliqué dans la régulation de l'homéostasie de la barrière cutanée.

Des échantillons de peau de patients atteints de rosacée ont démontré, après coloration immunohistochimique, des niveaux plus élevés d'expression de PAR-2 que ceux des sujets sains.

L'activation de PAR-2 a plusieurs effets sur la fonction barrière. En particulier, elle diminue l'adhérence intercellulaire via l'E-cadhérine, ce qui se traduit par une perméabilité accrue de la barrière épidermique.

Il a été montré que des peptides agonistes de PAR-2 retardent de manière significative la récupération d'imperméabilité de la barrière épidermique après une perturbation aiguë de la barrière dans la peau d'un modèle murin.

Ces données montrent que KLK-5 est impliquée dans la perturbation de la fonction barrière observée dans la rosacée, notamment par le biais de son activation de PAR-2. De plus, l'implication de cette enzyme protéolytique (ou protéase) dans l'activation de LL-37 confirme son rôle majeur dans le processus inflammatoire qui accompagne les manifestations de la rosacée.

KLK-5 est donc une cible préférentielle pour le traitement de la rosacée et/ou de la télangiectasie car l'inhibition de son activité et/ou la diminution de son expression peut contribuer à renforcer la fonction barrière, ce qui limite la pénétration d'allergènes, de bactéries et d'agents irritants et, en même temps, réduire l'inflammation.

Le ciblage de la KLK-5 par des actifs dermatologiques a déjà été décrit. A titre d'exemple, le document FR 3 022 139 décrit une composition cosmétique comprenant des sels minéraux de la gomme xanthane, un extrait hydroalcoolique de ginseng et un extrait d'*Ascophyllum nodosum,* pour son utilisation dans le traitement de la rosacée par le biais de son activité inhibitrice de l'activité enzymatique de la KLK-5.

Cependant, cette composition cosmétique présente l'inconvénient majeur de contenir des extraits non-titrés en molécules actives et dont la composition n'apparait ni connue ni maitrisée.

D'autres traitements conventionnels consistent à administrer au patient des antibiotiques pour leur effet anti-inflammatoire. Toutefois, de tels traitements sont photosensibilisants (augmentation de la sensibilité de la peau aux UV) et nécessitent l'utilisation d'une protection solaire efficace. Des traitements locaux (crème, gel ou émulsion) sont également connus, éventuellement en combinaison avec l'administration orale d'antiobiotiques. Cependant, ces traitements ont pour inconvénients d'entrainer des effets indésirables chez le sujet atteint de rosacée, comme une aggravation des rougeurs ou une sensation d'échauffement de la peau.

Un autre traitement de la rosacée disponible consiste à recourir au laser afin de faire éclater les petits vaisseaux, par photothermolyse sélective ou à souder les petits vaisseaux par photothermocoagulation. Un inconvénient majeur de ce traitement est qu'il est coûteux. En outre, il nécessite d'être effectué en dehors des périodes ensoleillées.

Il subsiste donc un besoin évident de mettre au point une composition comprenant des composés actifs dermatologiques qui soient surs pour l'utilisation topique humaine et permettant de traiter efficacement et à long terme la rosacée et/ou la télangiectasie à travers l'inhibition de la surproduction de la protéase KLK-5.

### Description de l'invention

Le Demandeur a constaté que, de manière surprenante, plusieurs principes actifs dermatologiques, qui sont parfaitement sûrs et adaptés à l'utilisation cosmétique humaine, permettent de surmonter les inconvénients et de répondre aux besoins précédemment décrits.

L'invention vise, de manière générale, à répondre à un besoin cosmétique réel en hydratant et apaisant la peau, en réduisant les sensations d'inconfort et, *in fine,* en améliorant l'aspect de la peau.

En particulier, l'invention vise à traiter la rosacée et/ou la télangiectasie afin d'améliorer l'aspect de la peau, réduire les imperfections.

Un but de l'invention est de proposer une composition permettant de diminuer le clivage de la protéine hCAP 18 en LL-37, diminuer la teneur en LL-37 dans la peau, diminuer l'expression du gène de KLK-5 et/ou l'activité protéolytique de l'enzyme KLK-5 et diminuer l'expression du gène de PAR-2 et/ou l'activité de PAR-2.

En particulier, le Demandeur a constaté que les principes actifs dermatologiques utilisés pour l'invention permettent de réduire les niveaux de production de KLK-5 et/ou d'expression du gène de la protéase KLK-5 et/ou l'activité protéolytique de KLK-5 et de ce fait, l'invention permet de traiter l'inflammation cutanée et/ou de renforcer la fonction barrière de la peau.

Au sens de l'invention, par « principe actif dermatologique » on désigne une substance ou un composé destiné à être appliqué sur la peau et qui possède des propriétés biologiques et/ou thérapeutiques qui sous-tendent un effet physiologique.

Dans la suite de la description, par « composition selon l'invention » ou « composition de l'invention » on désigne la composition pour utilisation pour le traitement de la rosacée et/ ou de la télangiectasie telle que définie dans la revendication 1.

Le principe actif dermatologique est à différencier du au moins un excipient, présent dans la composition selon l'invention.

Au sens de l'invention, par « niveaux de KLK-5 » on désigne le niveau de production de la protéase KLK-5, c'est-à-dire la teneur en KLK-5, et/ou le niveau d'expression du gène de la protéase KLK-5 et/ou l'activité de la protéase KLK-5. Au sens de l'invention, par « excipient » on désigne une autre substance que le principe actif dermatologique qui confère à la composition des propriétés, notamment, de consistance, galénique et/ou de vectorisation du principe actif.

Ainsi et selon un premier aspect, l'invention concerne une composition comprenant, en tant que principe actif dermatologique, au moins un polysaccharide consistant en :
- un hétéropolymère de glucose et tréhalose correspondant à la désignation INCI maltooligosyl glucoside ; et
- un homopolymère hydrogéné de glucose ramifié comprenant des liaisons alpha (1-4) et alpha (1-6) correspondant à la désignation INCI hydrogenated starch hydrolysate,
pour utilisation pour le traitement de la rosacée et/ou de la télangiectasie.

Dans la composition selon l'invention, l'hétéropolymère de glucose et tréhalose correspond à la désignation INCI maltooligosyl glucoside (ou maltooligosyl glucoside (INCI)). Dans la suite de la description, par « maltooligosyl glucoside » on entend la désignation INCI maltooligosyl glucoside.

Au sens de l'invention, le maltooligosyl glucoside correspond à un mélange d'oligosaccharides constitués d'un, deux, trois ou quatre résidus de glucose lié à un résidu de tréhalose.

Selon un mode de réalisation particulier, le maltooligosyl glucoside (INCI) comprend du maltotriosyl glucoside, c'est-à-dire, un oligosaccharide constitué de trois résidus de glucose liés à un résidu de tréhalose.

Selon un autre mode de réalisation particulier, le maltooligosyl glucoside (INCI) est un mélange de plusieurs oligosaccharides comprenant du maltotriosyl glucoside dans une quantité comprise entre 40% et 90% en poids total du mélange d'oligosaccharides (c'est-à-dire en poids total du maltooligosyl glucoside), avantageusement entre 50% et 80%.

Selon un autre mode de réalisation particulier, le maltooligosyl glucoside (INCI) comprend la combinaison de maltriosyl glucoside et de maltotetrasyl glucoside, ladite combinaison représentant entre 10% et 40% en poids total du mélange d'oligosaccharides (c'est-à-dire en poids total du maltooligosyl glucoside), de préférence entre 15% et 25%.

Avantageusement, le maltooligosyl glucoside (INCI) comprend, en outre, du maltotetraitol et maltotriiol.

Le maltooligosyl glucoside (INCI) peut être obtenu par synthèse chimique ou, de préférence, par biotechnologie.

Selon un mode de réalisation particulier, le maltooligosyl glucoside représente entre 0,001% et 2% en poids de la composition, avantageusement entre 0,01% à 0,5%.

Dans la composition selon l'invention, l'homopolymère hydrogéné de glucose ramifié comprenant des liaisons α (1-4) et α (1-6) correspond à la désignation INCI hydrogenated starch hydrolysate (ou hydrogenated starch hydrolysate (INCI)). Dans la suite de la description, par « hydrogenated starch hydrolysate » on entend la désignation INCI hydrogenated starch hydrolysate.

L'hydrogenated starch hydrolysate (INCI) ou hydrolysat d'amidon hydrogéné, est obtenu par hydrogénation contrôlé de l'amidon végétale partiellement digéré. Il peut s'agir d'un amidon extrait d'un ou plusieurs végétaux, comme par exemple le maïs et/ou la pomme de terre. L'hydrolyse peut être effectuée, par exemple, par digestion enzymatique ou traitement avec un acide faible tandis que l'hydrogénation peut s'effectuer selon les méthodes connues à l'homme du métier dans le domaine de l'industrie alimentaire, par exemple, l'incubation avec du dihydrogène gazeux en présence de catalyseurs métalliques.

Selon un mode de réalisation particulier, l'hydrogenated starch hydrolysate représente entre 0,001% et 2% en poids de la composition, avantageusement entre 0,01% et 0,5%.

Selon un mode de réalisation particulier non revendiqué, l'homopolymère de glucose ramifiés comprenant des liaisons α (1-4) et α (1-6) correspond à la désignation INCI glycogen (ou glycogen (INCI)). Dans la suite de la description, par « glycogen » on entend la désignation INCI glycogen.

Selon un mode de réalisation particulier non revendiqué, l'homopolymère de glucose ramifié comprenant des liaisons α (1-4) et α (1-6), avantageusement le glycogen, représente entre 0,001% et 2% en poids de la composition, avantageusement entre 0,01% et 0,6%.

Selon un mode de réalisation particulier non revendiqué, l'homopolymère de glucose ramifié comprenant des liaisons α (1-4) et α (1-6), avantageusement le glycogen, a un poids moléculaire compris entre 1 KDa et 10 KDa, avantageusement entre 4 et 6 KDa.

Le glycogène est un polysaccharide de glucose ramifié qu'on retrouve dans la plupart des organismes, notamment chez l'Homme. L'homopolymère de glucose ramifié comprenant des liaisons α (1-4) et α (1-6), avantageusement le glycogen, peut être obtenu par synthèse chimique ou, de préférence, par biotechnologie.

L'homopolymère de glucose ramifié comprenant des liaisons α (1-4) et α (1-6), avantageusement le glycogen, peut être d'origine animale ou végétale, par exemple, issu de maïs ou de la pomme de terre, ou encore.

Avantageusement, le glycogen (INCI) correspond à du glycogène dont la pureté est de 80%, voire 90%, de préférence 95%, voire 99% ou plus, par exemple 100%.

Selon un mode de réalisation particulier non revendiqué, l'homopolymère de glucose ramifié comprenant des liaisons α (1-4) et α (1-6) selon l'invention correspondant à la désignation INCI glycogen (INCI) est la matière première BIOGLYCOGEN^{®} commercialisée par la société GLICO NUTRITION CO., LTD.

Selon un mode de réalisation préféré non revendiqué, la composition selon l'invention comprend :
- au moins un hétéropolymère de glucose et tréhalose, représentant avantageusement entre 0,001% et 2% en poids de la composition, de préférence entre 0,01% à 0,5% ; et
- au moins un homopolymère hydrogéné de glucose ramifiés comprenant des liaisons α-(1-4) et α-(1-6), représentant avantageusement entre 0,001% et 2% en poids de la composition, de préférence entre 0,01% et 0,5%.

La composition selon l'invention comprend du maltooligosyl glucoside et de l'hydrogenated starch hydrolysate.

Selon un mode de réalisation particulier, la composition selon l'invention comprend :
- au moins un hétéropolymère de glucose et tréhalose, représentant avantageusement entre 0,001% et 2% en poids de la composition, de préférence entre 0,01% à 0,5% ;
- au moins un homopolymère hydrogéné de glucose ramifiés comprenant des liaisons α-(1-4) et α-(1-6), représentant avantageusement entre 0,001% et 2% en poids de la composition, de préférence entre 0,01% et 0,5% ; et
- au moins un homopolymère de glucose ramifiés comprenant des liaisons α-(1-4) et α-(1-6), représentant avantageusement entre 0,001% et 2% en poids de la composition, avantageusement entre 0,01% et 0,6%.

Avantageusement, la composition selon l'invention comprend du maltooligosyl glucoside, de l'hydrogenated starch hydrolysate et du glycogen.

Selon un mode de réalisation particulier, la composition selon l'invention comprend :
- au moins un hétéropolymère de glucose et tréhalose, représentant avantageusement entre 0,001% et 2% en poids de la composition, de préférence entre 0,01% à 0,5% ;
- au moins un homopolymère hydrogéné de glucose ramifiés comprenant des liaisons α-(1-4) et α-(1-6), représentant avantageusement entre 0,001% et 2% en poids de la composition, de préférence entre 0,01% et 0,5% ; et éventuellement
- au moins un homopolymère de glucose ramifiés comprenant des liaisons α-(1-4) et α-(1-6), représentant avantageusement entre 0,001% et 2% en poids de la composition, avantageusement entre 0,01% et 0,6% ; en outre, ladite composition présente un rapport pondéral entre le au moins un hétéropolymère de glucose et tréhalose et le au moins un homopolymère hydrogéné de glucose ramifiés comprenant des liaisons α-(1-4) et α-(1-6), avantageusement entre le INCI maltooligosyl glucoside et le glycogen, compris entre 3/1 et 1/3, de préférence entre 2/1 et 1/2.

Selon un mode de réalisation particulier, la composition selon l'invention est une composition cosmétique.

Il ressort de ce qui précède que la composition, avantageusement cosmétique, selon l'invention permet de soulager la gêne et l'inconfort lié à la rosacée et/ou la télangiectasie. En particulier, la composition selon l'invention réduit l'intensité des rougeurs, apaise (réduction des tiraillements et des sensations de rugosité) et hydrate la peau. Ainsi, la présente invention permet de répondre à un besoin cosmétique réel.

Selon un mode de réalisation particulier, la composition selon l'invention est exempte de probiotiques.

Au sens de l'invention, par « probiotiques » on désigne des microorganismes vivants qui confèrent un bénéfice pour la santé de l'hôte, lorsqu'ils sont consommés à des concentrations suffisantes. Ils peuvent interagir avec l'hôte pour améliorer l'immunité, l'homéostasie intestinale, stimuler le métabolisme ou encore réduire le risque d'infection par des agents pathogènes opportunistes. La plupart des probiotiques bactériens appartiennent aux bactéries lactiques. Ce groupe inclut 10 genres dont les plus connus sont *Lactobacillus, Pediococcus, Lactococcus, Enterococcus, Streptococcus, Leuconostoc* et *Camobacterium.*

Avantageusement la composition selon l'invention est exempte des probiotiques suivants : *Lactobacillus casei, Lactobacillus acidophillus, Bacillus coagulans* et/ou Bifida ferment lysate (INCI).

Selon un autre mode de réalisation, la composition selon l'invention comprend, en outre, au moins un filtre UV. La filtration de la lumière UV est connue pour améliorer l'état générale de la peau, notamment chez les individus atteints par la rosacée et/ou la télangiectasie.

Au sens de l'invention, par « filtre UV » on désigne les composés organiques (ou composés chimiques) ou minéraux capables de filtrer les UV-A, les UV-B et/ou les UV-C.

Selon l'invention, il peut aussi bien s'agir de filtres minéraux que de filtres chimiques ou organiques.

Les compositions selon l'invention peuvent contenir un ou plusieurs filtres UV à large spectre, c'est-à-dire des composés ou des mélanges qui absorbent les UV-A, les UV-B, les UV-C et éventuellement la lumière visible.

Parmi les filtres organiques à large spectre, les filtres correspondant aux désignations INCI suivantes peuvent être utilisés dans le cadre de l'invention : tris biphenyl triazine, bis ethylhexyloxyphenol methoxyphenyl triazine, methylene bis-benzotriazolyl tetramethylbutylphenol. A titre d'exemple, ceux-ci sont commercialisés par la société BASF sous les noms de TINOSORB S^{®}/TINOSORB AQLIA^{®}, TINOSORB A2B^{®}, TINOSORB M^{®}, respectivement. Un autre exemple de filtre à large spectre adapté à la composition selon l'invention répond à la désignation INCI diethylhexyl butamido triazone, par exemple commercialisé par la société SIGMA 3V sous le nom d'UVASORB HEB^{®}.

Ainsi et dans un mode de réalisation particulier, la composition selon l'invention comprend au moins un filtre choisi dans le groupe suivant de composés identifiés par leur désignation INCI : tris biphenyl triazine, bis ethylhexyloxyphenol methoxyphenyl triazine, et methylene bis-benzotriazolyl tetramethylbutylphenol, diethylhexyl butamido triazone, ou leurs mélanges.

Avantageusement, la composition comprend le filtre bis-ethylhexyloxyphenol methoxyphenyl triazine.

Selon un autre mode de réalisation, à la place ou en plus du ou des filtre(s) à large spectre, la composition contient au moins un filtre UV-A et/ou UV-B, organique et/ou minéral, qui peut se présenter en phase aqueuse (lipophile) et/ou huileuse (liposoluble).

Ainsi et à titre d'exemple, la composition selon l'invention peut contenir des filtres UV-B liposolubles, susceptibles de contribuer à la stabilisation ou à la solubilisation des filtres à large spectre ou encore de se stabiliser réciproquement et par ce fait, augmenter le facteur de protection solaire (FPS ou « SPF » en anglais).

Avantageusement, de tels filtres correspondent aux désignations INCI suivantes: homosalate, octocrylene, ethylhexyl salicylate, ethylhexyl triazone.

Dans un mode de réalisation préféré, la composition selon l'invention comprend de l'ethylhexyl triazone, commercialisé par BASF sous le nom d'UVINUL T150^{®}.

Dans un autre mode de réalisation, le filtre UV-B liposoluble est l'α-(triméthylsilyl)-ω-(triméthylsilyloxy)poly[oxy(diméthyl)silylène]-co-[oxy(méthyl)(2-{4-[2,2-bis(éthoxycarbonyl)vinyl]phénoxy}-1-méthylèneéthyl)silylène]-co-[oxy(méthyl)(2-(4-[2,2-bis(éthoxycarbonyl)vinyl]phénoxy)prop-1-ényl)silylène], un polymère siliconé capable de filtrer dans l'UV-B. Ce filtre correspond par exemple à la matière première cosmétique Parsol SLX^{®}, commercialisée par la société DSM selon la désignation INCI polysilicone-15.

Dans un mode de réalisation particulier, la composition selon l'invention comprend au moins un filtre UV-B choisi dans le groupe suivant de composés identifiés par leur désignation INCI : homosalate, ethylhexyl salicylate, ethylhexyl triazone, polysilicone-15, ou leurs mélanges.

Dans un mode de réalisation particulier, la composition est exempte des filtres suivants : 4-methylbenzylidene camphor, benzophenone-2, benzophenone-3, ethylhexyl methoxycinnamate, octocrylene.

Dans un mode de réalisation avantageux, la composition selon l'invention comprend au moins un filtre UV-A, afin d'assurer une filtration complète de la partie nuisible du spectre solaire.

Des filtres UV-A avantageux au sens de la présente invention sont le butyl methoxydibenzoylmethane (INCI) et le diethylamino hydroxybenzoyl hexyl benzoate (INCI), correspondant respectivement aux matières premières Parsol 1789^{®} commercialisée par la société DSM et UVINLIL^{®} A+ commercialisée par la société BASF.

Dans un mode de réalisation particulier, le filtre UV-A est le bis-(diethylaminohydroxybenzoyl benzoyl) piperazine (INCI) (numéro CAS 919803-06-8), correspondant par exemple à la matière première C1332^{®} commercialisée par la société BASF.

Ainsi et dans un mode de réalisation préféré, la composition selon l'invention comprend au moins un filtre choisi dans le groupe suivant de composés identifiés par leur désignation inci : butyl methoxydibenzoylmethane, diethylamino hydroxybenzoyl hexyl benzoate, bis- (diethylaminohydroxybenzoyl benzoyl) piperazine, ou leurs mélanges.

D'autres filtres UV avantageux au sens de la présente invention sont des filtres hydrosolubles, comme par exemple :
- le filtre correspondant à la désignation INCI disodium phenyl dibenzimidazole tetrasulfonate, notamment disponible sous la dénomination Neo Heliopan^{®} AP (Symrise) ;
- le filtre correspondant à la désignation INCI phenylbenzimidazole sulfonic acid, notamment disponible sous la dénomination Neo Heliopan^{®} hydro (Symrise), de préférence en combinaison avec un acide aminé basique, avantageusement de l'arginine. En pratique, l'acide aminé basique représente entre 0,5 et 2% en poids de la composition, de préférence entre 1 et 1,5% en poids de la composition.

Dans un mode de réalisation préféré, la composition selon l'invention comprend au moins un filtre hydrosoluble choisi dans le groupe suivant de composés identifiés par leur désignation INCI : disodium phenyl dibenzimidazole tetrasulfonate, phenylbenzimidazole sulfonic acid, ou leurs mélanges.

Avantageusement, les filtres minéraux inorganiques, ou écrans minéraux, sont des oxydes métalliques et/ou d'autres composés difficilement solubles ou insolubles dans l'eau, en particulier les oxydes de titane (TiO₂), de zinc (ZnO), de fer (Fe₂O₃), de zirconium (ZrO₂), de silicium (SiO₂), de manganèse (par exemple MnO), d'aluminium (Al₂O₃), ou de cérium (Ce₂O₃).

Selon un mode de réalisation particulier, les filtres minéraux inorganiques peuvent être utilisés sous forme de prédispersion huileuse ou aqueuse disponible sur le marché. Ces prédispersions peuvent être additionnées avantageusement d'auxiliaires de dispersion et/ou de médiateurs de solubilisation.

Les filtres minéraux inorganiques peuvent également être traités en surface ou encapsulés, afin de leur conférer un caractère hydrophile, amphiphile ou hydrophobe. Ce traitement de surface peut consister en ce que les filtres minéraux soient dotés d'une mince pellicule inorganique et/ou organique hydrophile et/ou hydrophobe.

Dans un mode de réalisation préféré, la composition selon l'invention comprend au moins un écran minéral choisi dans le groupe suivant de composés identifiés par leur désignation INCI : Zinc oxide, Titanium dioxide, ou leurs mélanges.

Les listes des filtres UV cités pouvant être mis en oeuvre au sens de la présente invention sont bien entendu donnés à titre indicatif et non limitatif.

De manière avantageuse, les filtres UV tels que décrits ci-dessus, présents dans la composition selon l'invention, représentent de 0,1% à 30% en poids de la composition, avantageusement entre 0,5 % et 20 %, encore plus avantageusement entre 1% et 15%.

Selon un mode de réalisation particulier, la composition selon l'invention présente un facteur de protection solaire (« SPF » ou FPS) supérieur ou égal à 10, de préférence supérieur ou égal à 20, avantageusement supérieur ou égal à 30, encore plus avantageusement supérieur ou égal à 50.

Selon un mode de réalisation préféré, la composition selon l'invention comporte un ratio de protection UV-A/UV-B égal ou supérieur à 1/3.

La composition solaire selon l'invention comprend au moins un solubilisants de filtres solaires choisi dans le groupe suivant de composés identifiés par leur désignation INCI : caprylyl caprylate/caprate, dibutyl adipate, dicaprylyl carbonate, diisopropyl sebacate, dicaprylyl ether, coco-caprylate, C12-15 alkyl benzoate, propylheptyl caprylate et butylene glycol dicaprylate/dicaprate. Ces solubilisants sont disponibles sur le marché auprès de plusieurs fournisseurs. A titre d'exemple, les matières premières suivantes peuvent être mises en oeuvre dans la composition selon l'invention :
- Plusieurs matières premières de la gamme CETIOL^{®} commercialisées par la société BASF, notamment le CETIOL^{®} RLF, CETIOL^{®} B, CETIOL^{®} CC, CETIOL^{®} O, CETIOL^{®} C5, CETIOL^{®} AB, CETIOL^{®} SENSOFT correspondant respectivement aux désignations INCI caprylyl caprylate/caprate, dibutyl adipate, dicaprylyl carbonate, dicaprylyl ether, coco-caprylate, C12-15 alkyl benzoate, propylheptyl caprylate ;
- le DUB DIS commercialisé par la société STEARINE DUBOIS correspondant à la désignation INCI diisopropyl sebacate ;
- Le MIGLYOL^{®} 8810 commercialisé par la société IOI Oleo GmbH correspondant à la désignation INCI butylène glycol dicaprylate/dicaprate.

Dans un mode de réalisation préféré, la composition selon l'invention comprend au moins quatre solubilisants choisi dans le groupe suivant de composés identifiés par leur désignation INCI : caprylyl caprylate/caprate, dibutyl adipate, dicaprylyl carbonate, diisopropyl sebacate, dicaprylyl ether, coco-caprylate, C12-15 alkyl benzoate, propylheptyl caprylate et butylene glycol dicaprylate/dicaprate

Dans un mode de réalisation particulier, la composition selon l'invention comprend les solubilisants répondant aux désignations INCI dibutyl adipate, dicaprylyl carbonate, diisopropyl sebacate. Dans un mode de réalisation préféré, la composition selon l'invention comprend en outre au moins un autre solubilisant choisi dans le groupe suivant de composés identifiés par leur désignation INCI : propylheptyl caprylate, dicaprylyl ether, coco-caprylate, C12-15 alkyl benzoate, caprylyl caprylate/caprate. Avantageusement, il s'agit du propylheptyl caprylate.

Selon un mode de réalisation préféré, le poids total des solubilisants tels que définis ci-dessus représentent entre 5% et 80% en poids sur le total de la composition, avantageusement entre 10% et 70%, encore plus avantageusement entre 15 et 60%.

La composition selon l'invention, peut en outre comprendre un « booster » de SPF, c'est-à-dire un agent amplificateur du facteur de protection solaire, et/ou un photostabilisant, c'est à dire un ingrédient qui permet d'augmenter le SPF ou de photostabiliser les filtres, un tel ingrédient n'étant pas considéré lui-même comme un filtre solaire. On peut par exemple citer :
- le butyloctyl salicylate (INCI), photostabilisant représentant avantageusement entre 0,01% et 10% en poids de la composition, encore plus avantageusement ente 0,1% et 2%. Cette matière première est par exemple commercialisée par la société HALLSTAR sous le nom de Hallbrite^{®} BHB;
- le benzotriazolyl dodecyl p-cresol (INCI), photostabilisant représentant avantageusement entre 0,01% et 10% en poids de la composition, encore plus avantageusement entre 0,1% et 2%. Cette matière première est par exemple commercialisée par la société BASF sous le nom de TINOGARD^{®} TL;
- le pongamol (INCI), molécule végétale absorbant dans les UV-A, représentant avantageusement entre 0,5 et 2% en poids de la composition, encore plus avantageusement de l'ordre de 1%. A titre d'exemple, la matière première Pongamia Extract commercialisée par la société GIVAUDAN peut être utilisée dans le cadre la présente invention ;
- l'ethylhexyl methoxycrylene (INCI), photostabilisant, solubilisant et « booster » de SPF représentant avantageusement entre 1% et 5% en poids de la composition. La matière première SolaStay^{®} S1 commercialisée par la société HALLSTAR peut être utilisée dans le cadre de la présente invention ;
- un copolymère de styrène acrylate (INCI : styrene/acrylate copolymer), représentant de préférence entre 1% et 10% en poids de la composition selon l'invention. Les matières premières SunSpheres^{®} H53 et SunSpheres^{®} PGL Polymer, commercialisées par la société DOW CHEMICALS, peuvent être utilisées dans le cadre de la présente invention ;
- le diethylhexyl syringylidene malonate (INCI), représentant avantageusement entre 1% et 10% en poids de la composition. La matière première OXYNET^{®} ST, commercialisée par la société MERCK, peut être utilisée dans le cadre de la présente invention ;
- un polyester hydrodispersible, correspondant aux désignations INCI polyester-5 (and) Sodium silicoaluminate, représentant avantageusement entre 1% et 10% en poids de la composition, notamment l'EASTMANN AQTM38S Polymer commercialisé par la société SAFIC-ALCAN ;
- un copolymère d'acrylate ayant une température de transition vitreuse de -5°C à
- 15°C telle que mesurée par calorimétrie différentielle à balayage, ledit copolymère représentant avantageusement entre 1% et 10% en poids de la composition. Par exemple, un polymère correspondant à la désignation INCI Acrylate copolymer, tel que la matière première EPITEX 66, commercialisée par la société DOW CHEMICALS, peut être utilisée dans le cadre de la présente invention.

Dans un mode de réalisation particulier, la composition selon l'invention comprend également une ou plusieurs substances aptes à filtrer la lumière visible, en particulier la lumière bleue. A titre d'exemple, les composés décrits dans le document EP 1 484 051 peuvent être employés pour assurer une filtration de la lumière bleue.

Selon un autre aspect de l'invention, les compositions selon l'invention sont formulées à partir d'une base aqueuse ayant une composition définie et contenant notamment du chlorure de calcium ; du chlorure de potassium ; du phosphate de potassium ; du sulfate de magnésium ; du chlorure de sodium ; de l'hydrogénocarbonate de sodium ; de l'hydrogénophosphate de sodium ; du citrate de sodium ; de l'acide citrique ; de la carnosine et de l'hypotaurine (acide 2-aminoéthanesulfinique).

De préférence, ladite base aqueuse comprend :
- De 0,000001 % à 0,1 % en poids total de la base aqueuse de chlorure de calcium, avantageusement de 0,0001% à 0,1 % ;
- De 0,0001% à 0,1% en poids total de la base 5 aqueuse de chlorure de potassium, avantageusement de 0,001% à 0,1% ;
- De 0,00001% à 0,1% en poids total de la base aqueuse de phosphate de potassium, avantageusement de 0,0001% à 0,1 % ;
- De 0,0001% à 0,1 % en poids total de la base aqueuse de sulfate de magnésium, avantageusement de 0,001% à 0,1%; - De 0,001 à 4% de en poids total de la base aqueuse chlorure de sodium, avantageusement de 0,1 à 4% ;
- De 0,0001 à 0,1% en poids total de la base aqueuse d'hydrogénocarbonate de sodium ; - De 0,0001% à 0,1% en poids total de la base aqueuse d'hydrogénophosphate de sodium ;
- De 0,0001% à 1% en poids total de la base aqueuse d'acide citrique, avantageusement de 0,001% à 1 % ;
- De 0,001% à 1% en poids total de la base aqueuse de citrate de sodium ;
- De 0,0001% à 1% en poids total de la base aqueuse de carnosine, avantageusement de 0,001% à 1 % ;
- De 0,0001 à 1% en poids total de la base aqueuse d'acide 2-aminoéthanesulfinique, avantageusement de 0,001 à 1%.

Dans un mode de réalisation particulier, la composition selon l'invention contient également des principes actifs aptes à améliorer l'hydratation cutanée.

Dans un mode de réalisation particulier, la composition avantageusement cosmétique selon l'invention comprend en outre, un extrait de pépins de pomme, éventuellement complémenté par de la vitamine B3 (niacinamide ou vitamine PP) ou ses dérivés. L'extrait de pépins de pomme (*Pyrus malus*) permet d'augmenter la synthèse de plusieurs aquaporines. Les aquaporines sont des protéines qui jouent un rôle clef dans les équilibres de l'hydratation cutanée.

La vitamine PP et ses dérivés agissent en stimulant l'activité de la sérine palmitoyl transférase, une enzyme impliquée dans la synthèse de sphingosine, molécule précurseur des céramides et de ce fait, améliorent la fonction barrière de la peau et combattent la sécheresse cutanée.

De préférence, l'extrait de pépins de pomme est un extrait de nature lipophile. En pratique, la matière première cosmétique EDERLINE S, correspondant à la désignation INCI Hexyldecanol & butylene glycol & *Pyrus malus* (apple) seed extract, ou encore la matière première EDERLINE LS, correspondant à la désignation INCI hexyldecanol & *Pyrus malus* (apple) seed extract & *Brassica campestris* (rapeseed) sterols & tocopherol, peuvent être utilisées comme source d'extrait de pépins de pommes. Quant à la matière première niacinamide, elle est commercialisée par l'entreprise QUIMICA MASSO et correspond à la désignation INCI niacinamide.

Avantageusement, la composition selon l'invention comprend de 0,001% à 1% d'extrait végétal de *Pyrus malus* et/ou de 0,001% à 10% de niacinamide en poids total de la composition.

Dans un autre mode de réalisation, la composition avantageusement cosmétique selon l'invention comprend en outre, un extrait de la plante *Imperata cylindrica* (*I. cylindrica*), de préférence un extrait de racine, avantageusement titré en aquaporines. En effet, l'incorporation d'extraits d*'Imperata cylindrica* dans la composition comprenant la composition selon l'invention permet d'améliorer l'hydratation du *stratum corneum.*

En pratique, la matière première MOIST 24 PH correspondant à la désignation INCI *I*. *cylindrica* root extract & water & glycerin & PEG-8 & carbomer & phenoxyethanol & sodium citrate & potassium sorbate & citric acid et commercialisée par la société SEDERMA peut être utilisée dans le cadre de la présente invention. Avantageusement, la composition selon l'invention comprend entre 0,001% et 1% en poids de ladite composition d'extrait végétal d'*I*. *cylindrica,* de préférence entre 0,01% et 0,1 %.

Dans un autre mode de réalisation, la composition avantageusement cosmétique selon l'invention comprend en outre, un système bioactif associant d'une part, une forme stable en solution aqueuse d'un nucléotide choisi parmi l'ATP (adenosine triphosphate) le Gp4G (diguanosine tetraphosphate) et l'Ap4A (diadénosine tetraphosphate) ; et d'autre part, au moins un peptide biomimétique comprenant au plus six acides aminés, mimant un polypeptide cutané ou une protéine cutanée, ou une biomolécule agoniste ou antagoniste dudit peptide ou de ladite protéine. En pratique, l'association de ces principes actifs permet de catalyser l'activité métabolique des cellules de la peau tout en obtenant un effet dermocosmétique ou thérapeutique grâce à l'utilisation des peptides biomimétiques. Ces derniers peuvent être sélectionnés afin d'obtenir l'effet souhaité, par exemple, un effet d'inhibition des irritations d'origine neurogène, une activité dépigmentante, un effet inhibant toute intolérance ou sensibilisation etc.

En pratique, dans le système bioactif selon l'invention, le nucléotide représente au plus 10% en poids de la composition, de préférence entre 0,001% et 5% ; et le peptide biomimétique représente entre 0,001% à 1 % en poids de la composition.

Selon un autre mode de réalisation, la composition selon l'invention peut comprendre en outre, un extrait de la bactérie *Arthrobacter agilis,* notamment un extrait riche en caroténoïdes. Ainsi la matière première correspondant à la désignation INCI micrococcus lysate commercialisée par la société GREENTECH peut être utilisée dans le cadre de la présente invention. Avantageusement, la composition selon l'invention comprend entre 0,00001% et 0,1% en poids de la composition, de préférence entre 0,0001% et 0,001% d'un tel extrait sec.

Selon un autre mode de réalisation, la composition selon l'invention comprend en outre d'autres composants pouvant contribuer à la protection interne par une action qui peut consister en une protection de l'ADN, une diminution de l'immunosuppression induite par les radiations UV, une action antiradicalaire ou un effet combiné de ces actions.

L'action protectrice d'une composition selon l'invention contre le stress oxydatif ou à l'encontre de l'effet des radicaux libres peut être encore améliorée si celle-ci comprend en outre un ou plusieurs antioxydants, aisément sélectionnées par l'Homme du métier par exemple dans la liste suivante : le totarol, le magnolol, l'honokiol, les acides aminés et leurs dérivés, des peptides et leurs dérivés (par exemple l'ansérine, l'hypotaurine, la taurine), les caroténoïdes, les carotènes (α-carotène, β-carotène, lycopène) et leurs dérivés, l'acide chlorogénique et ses dérivés, l'acide lipoïque et ses dérivés (acide dihydrolipoïque), l'aurothioglucose, le propylthiouracile et autres thiols (thiorédoxine, glutathion, cystéine, cystine, cystamine et leurs esters glycosyle, N-acétyle, méthyle, éthyle, propyle, amyle, butyle et lauryle, palmitoyle, oléyle, γ-linoléique, cholestéryle et glycéryle) ainsi que des sels de ceux-ci, le thiodipropionate de dilauryle, le thiodipropionate de distéaryle, l'acide thiodipropionique et ses dérivés, les composés sulfoximine (sulfoximine de buthionine, l'homocystéine sulfoximine, les buthionine sulfones, penta-, hexa- et heptathionine sulfoximine), des agents chélatants (tels que les acides α-hydroxygras, l'acide palmitique, l'acide phytique, la lactoferrine), les α-hydroxyacides (tels que l'acide citrique, lactique, ou malique), l'acide humique, l'acide biliaire, la bilirubine, la biliverdine, le tétraméthylène phosphonate pentasodique d'éthylènediamine et ses dérivés, les acides gras insaturés et leurs dérivés, la vitamine A et ses dérivés (palmitate de vitamine A), le benzoate de coniféryle, l'acide rutinique et ses dérivés, l'α-glycosyl rutine, l'acide férulique et ses dérivés, le furfurylideneglucitol, le butylhydroxytoluène, le butylhydroxyanisole, l'acide nordihydroguaiarétique, trihydroxybutyrophenone, la quercétine, l'acide urique et ses dérivés, le mannose et les dérivés de ceux-ci, le le sélénium et ses dérivés (sélénométhionine), les stilbènes et leurs dérivés (l'oxyde de stilbène, l'oxyde trans-stilbène).

Dans un mode de réalisation particulier, la composition selon l'invention contient en outre de l'acide glycyrrhétinique, un dérivé ou un sel de cet acide, utilisé comme apaisant (agent anti-inflammatoire) et représentant entre 0,01% et 2% en poids de la composition, de préférence entre 0,1% et 1%.

Selon un autre mode de réalisation de l'invention, la composition avantageusement cosmétique comprend au moins un, voire tous les constituants suivants exerçant une activité biologique in vivo sur les cellules de la peau, des lèvres, des cheveux et/ou des muqueuses soumises à un rayonnement UV-A et/ou UV-B, respectivement :
- un agent antiradicalaire préservant les structures cellulaires, tel que par exemple la vitamine E et/ou ses dérivés liposolubles ou hydrosolubles, en particulier le tocotriénol et/ou le tocophérol, représentant avantageusement entre 0,001% et 10% en poids de la composition, encore plus avantageusement entre 0,02% et 2%, de préférence 0,04% ;
- un agent limitant l'immunosuppression, tel que par exemple la vitamine PP, représentant avantageusement entre 0,001% et 1% en poids de la composition, de préférence entre 0,01% et 0,3%;
- un agent protecteur de la protéine p53, tel que par exemple l'épigallocatéchine gallate (EGCG), représentant avantageusement entre 0,001% et 0,1% en poids de la composition, de préférence entre 0,005% et 0,05%.

Dans un mode de réalisation particulier de l'invention, la composition avantageusement cosmétique selon l'invention contient également des principes actifs aptes à stimuler la prolifération de cellules cutanées, avantageusement des fibroblastes et/ou de kératinocytes, de préférence des fibroblastes humains ou animaux. En particulier et selon ce mode de réalisation, la composition cosmétique selon l'invention comprend en outre :
- de l'acide α-lipoïque ou l'un de ses sels ;
- un dérivé de vitamine C choisi parmi l'éthyle acide ascorbique et l'ascorbate de sodium ou leur mélange ; et
- de l'acide hyaluronique, dont le poids moléculaire (Mw) est avantageusement compris entre 0,5 et 15 kDa, de préférence 0,5 et 10 kDa.

Avantageusement, l'acide α-lipoïque ou l'un de ses sels représente de moins de 0,1%, avantageusement moins de 0,01% en poids du milieu de culture cellulaire, de préférence entre 0,001% et 0,0005%.

Avantageusement, le dérivé de vitamine C choisi parmi l'éthyle acide ascorbique et l'ascorbate de sodium ou leur mélange, avantageusement l'éthyle acide ascorbique, de préférence le 3-O éthyl éther d'acide ascorbique, représente entre 0,001% et 10% en poids du milieu de culture cellulaire, avantageusement entre 0,01% et 5%, de préférence entre 0,1 % et 1 %.

Avantageusement, l'acide hyaluronique, dont le poids moléculaire (Mw) est avantageusement compris entre 0,5 et 15 kDa, de préférence 0,5 et 10 kDa, représente entre 0,001% et 10% en poids du milieu de culture cellulaire, avantageusement entre 0,01% et 5%, de préférence entre 0,1% et 1%.

La composition selon l'invention peut également comprendre en outre des extraits peptidiques de soja et/ou de blé.

En pratique, les extraits peptidiques proviennent de graines de soja et de blé sont issu d'une hydrolyse enzymatique desdites graines par l'intermédiaire de peptidases qui permet de récupérer des peptides d'une taille moyenne de 700 Daltons. Préférentiellement, l'extrait peptidique de soja est l'extrait identifié sous le numéro CAS 68607-88-5 de même que l'extrait peptidique de blé est l'extrait identifié sous le numéro CAS 70084-87-6. Les extraits de blé et soja peuvent correspondre aux désignations INCI Hydrolyzed wheat protein et Hydrolyzed soy protein, respectivement.

Dans un mode de réalisation particulier, les extraits peptidiques de soja et de blé sont utilisés ensemble, par exemple dans un rapport pondéral respectivement compris entre 80/20 et 20/80, avantageusement compris entre 70/30 et 30/70, de préférence égal à 60/40.

Dans un mode de réalisation avantageux, les extraits peptidiques de soja et/ou de blé sont exempts de tripeptides synthétiques GHK (glycyl-histidyl-lysine ; INCI : Tripeptide-1). En pratique, les extraits peptidiques de soja et/ou blé représentent entre 0,01% et 20% en poids de la composition, avantageusement entre 0,1% et 10%, de préférence entre 0,2% et 0,7%.

La composition selon l'invention est avantageusement formulée pour être cosmétiquement acceptable, c'est-à-dire compatible avec la peau, les muqueuses, les cheveux et le cuir chevelu. De préférence, la composition de l'invention est une composition pour application cutanée ou à usage topique.

Par « composition pour application cutanée » ou « à usage topique » on désigne une composition compatible avec une application sur la peau, les muqueuses, les cheveux et/ou le cuir chevelu, de préférence la peau humaine.

La composition de l'invention peut se présenter sous toutes les formes galéniques appropriées pour une application topique, notamment sous forme de solution aqueuse, hydroalcoolique, organique ou huileuse ; de suspension ou de dispersion dans des solvants ou des corps gras, de type lotion ou sérum ; sous forme de dispersion vésiculaire ; sous forme d'émulsion eau dans huile (E/H), huile dans eau (H/E) ou multiple telle qu'une émulsion eau dans huile dans eau (E/H/E). L'émulsion peut être plus au moins épaisse et se présente sous forme de crème ou de lait; la composition de l'invention peut se présenter également sous forme de pommade, de gel, de bâtonnet solide, de produits anhydres pâteux ou solides, de mousse, notamment aérosol, de composition biphasique ou encore de composition pulvérisable.

La forme galénique de la composition et son mode de préparation, et par conséquent les excipients appropriés à la composition de l'invention, peuvent être choisis par l'Homme du métier sur la base de ses connaissances générales en fonction du type de composition recherchée.

En particulier et selon un mode de réalisation préféré, les compositions selon la présente invention comprennent une particule avantageusement colorée à effet flouteur ou un mélange de plusieurs particules à effet flouteur. Ces particules à effet flouteur sont également désignées « particules à effet soft-focus » ou encore « particules à effet de flou ».

Selon l'invention, par « particule à effet flouteur » on entend une particule, avantageusement colorée, destinée à donner plus de transparence au teint et un effet de flou. En particulier, la particule à effet flouteur permet à la composition qui la contient d'atténuer, par effet optique, le microrelief cutané, et en particulier les défauts cutanés tels que les taches, les rides, les ridules.

Plusieurs particules à effet soft-focus ou flouteur sont disponibles sur les marchés ; à titre d'exemple, on peut citer les matières premières de la gamme Ronasphere (MERCK) à base de substrat silice, notamment le RONASPHERE FLAWLESS, ainsi que plusieurs matières premières commercialisées par les entreprises JGC-C&C (gamme Coverleaf à base de substrat séricite ou séricite/talc), Nihon Koken (gamme Relief Color, Silséem à base de substrat mica/silice), ou Miyoshi Kasei (gamme PC-Bail à base de substrat silice).

Selon un mode de réalisation préféré, les compositions de l'invention comprennent, à titre de particule à effet flouteur, une particule composite comprenant de la séricite, de l'oxyde de fer brun, du dioxyde de titane et de la silice. De telles particules sont commercialisées par exemple sous les références COVERLEAF NS ou JS ou MF par la société CHEMICALS AND CATALYSTS.

La particule colorée à effet flouteur peut avoir une structure qui peut être par exemple de type microsphères de silice contenant de l'oxyde de fer, tel que celui commercialisé par la société MIYOSHI sous la référence PC BALL PC-LL-100 P, ce pigment étant constitué de microsphères de silice contenant de l'oxyde de fer jaune. Selon un mode préféré, on utilise en qualité de particule colorée à effet flouteur une particule composite ayant une structure de type séricite/oxyde de fer brun/dioxyde de titane/silice tel que celui commercialisé sous la référence COVERLEAF MF par la société CHEMICALS AND CATALYSTS.

Selon un autre mode de réalisation, la matière première GRANPOWDER USQ commercialisée par l'entreprise Grant Industries Inc et correspondant aux désignations INCI polymethylsilsesquioxane (and) HDI/Trimethylol Hexyllactone Crosspolymer peut être utilisée en qualité de particule à effet flouteur.

Alternativement, la matière première COVAMED PMMA 2 MUSI commercialisée par l'entreprise SENSIENT COSMETICS TECHNOLOGY correspondant aux désignations INCI Methyl méthacrylate crosspolymer and Silica peut être utilisée en qualité de particule à effet flouteur.

Selon un mode de réalisation alternatif, la matière première D-400 commercialisée par l'entreprise TOCHI correspondant aux désignations INCI HDI/trimethylol hexyllactone crosspolymer et Silica peut être utilisée en qualité de particule colorée à effet flouteur.

Selon encore un autre mode de réalisation, la matière première KSP-100 commercialisée par l'entreprise SHINETSU correspondant à la désignation INCI Vinyl dimethicone/methicone silsesquioxane crosspolymer peut être utilisée en qualité de particule à effet flouteur.

De préférence, les compositions selon l'invention comprennent de 0,5% à 20%, avantageusement de 1% à 10%, encore plus avantageusement de 1% à 5% en poids de particules à effet flouteur en poids de la composition.

Selon un mode de réalisation particulier, la composition selon l'invention comprend tout corps gras usuellement utilisé dans le domaine cosmétique. On peut notamment citer les corps gras siliconés tels que les huiles, les gommes et les cires de silicone, ainsi que les corps gras non siliconés tels que les huiles et les cires d'origine végétale, minérale, animale et/ou synthétique. Les huiles peuvent être volatiles ou non volatiles. On peut encore citer les hydrocarbures, les esters et les éthers de synthèse, les alcools gras et les acides gras. La composition peut également comprendre un milieu aqueux, un milieu hydroalcoolique contenant un alcool tel que l'éthanol ou l'isopropanol, ou un milieu organique comprenant des solvants organiques usuels tels que des alcools en C1-6, notamment l'éthanol et l'isopropanol, des glycols tels que le propylène glycol, des cétones.

La composition peut comprendre au moins un émulsifiant classique, choisi parmi les émulsifiants amphotères, anioniques, cationiques ou non ioniques, utilisés seuls ou en mélange.

Elle peut également comprendre les adjuvants habituels dans le domaine considéré, tels que les épaississants ou gélifiants hydrophiles ou lipophiles, les additifs hydrophiles ou lipophiles, les actifs notamment cosmétiques, les conservateurs, les antioxydants, les parfums, les charges, les pigments, les absorbeurs d'odeur, les colorants, les hydratants (glycérine), des vitamines, des acides gras essentiels, des polymères liposolubles notamment hydrocarbonés, les opacifiants, les stabilisants, les séquestrants, les conditionneurs et les agents propulseurs.

Bien entendu, l'homme du métier veillera à choisir ce ou ces éventuels adjuvants ou excipients complémentaires, et/ou leur quantité, de manière telle que les propriétés avantageuses de la composition selon l'invention ne soient pas, ou substantiellement pas, altérées par l'adjonction envisagée.

Il peut être particulièrement avantageux de formuler la composition de l'invention de façon qu'elle soit pulvérisable. Ceci peut être réalisé par exemple par la formulation d'émulsions spécifiques comprenant des combinaisons particulières d'excipients.

Avantageusement et en relation avec ces différentes applications, la composition de l'invention est appliquée ou administrée par voie topique.

La manière dont l'invention peut être réalisée et les avantages qui en découlent ressortiront mieux des exemples de réalisation qui suivent, donnés à titre indicatif et non limitatif, à l'appui des figures annexées.

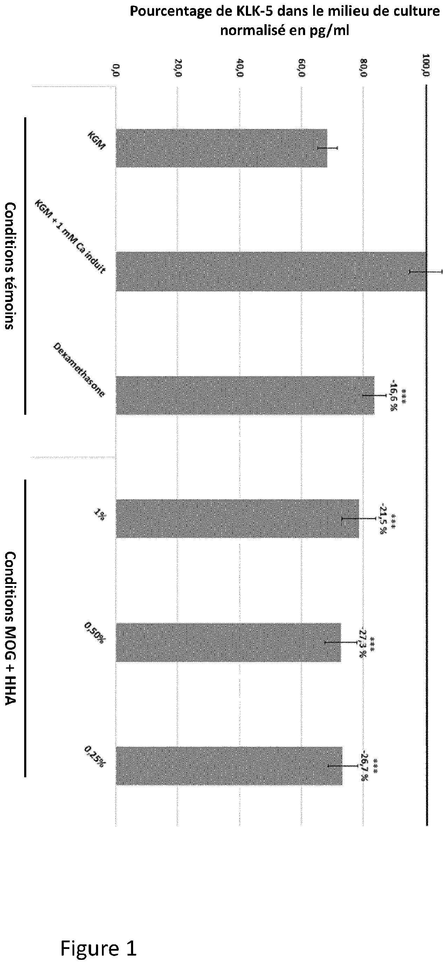

La figure 1 montre l'effet de l'association de polysaccharides maltooligosyl glucoside (MOG) et hydrolysat hydrogéné d'amidon (HHA) sur l'inhibition de la synthèse de KLK5 sécrétée par les KHN après 48 heures d'incubation. Le témoin positif choisi est la dexaméthasone à 1µM. Les résultats sont exprimés sous forme de pourcentage d'expression normalisée par rapport à la viabilité cellulaire. Ils représentent les données obtenues sur 1 expérience réalisée en triplicat.

### Description des figures

La figure 1 montre l'effet de l'association de polysaccharides maltooligosyl glucoside (INCI ; dénommé ci-après MOG) et hydrogenated starch hydrolysate (INCI ; dénommé ci-après HHA) sur l'inhibition de la synthèse de KLK-5 sécrétée par les kératinocytes humains normaux (KHN) après 48 heures d'incubation. Le témoin positif choisi est la dexaméthasone à 1µM. Les résultats sont exprimés sous forme de pourcentage d'expression normalisée par rapport à la viabilité cellulaire. Ils représentent les données obtenues sur 1 expérience réalisée en triplicate.

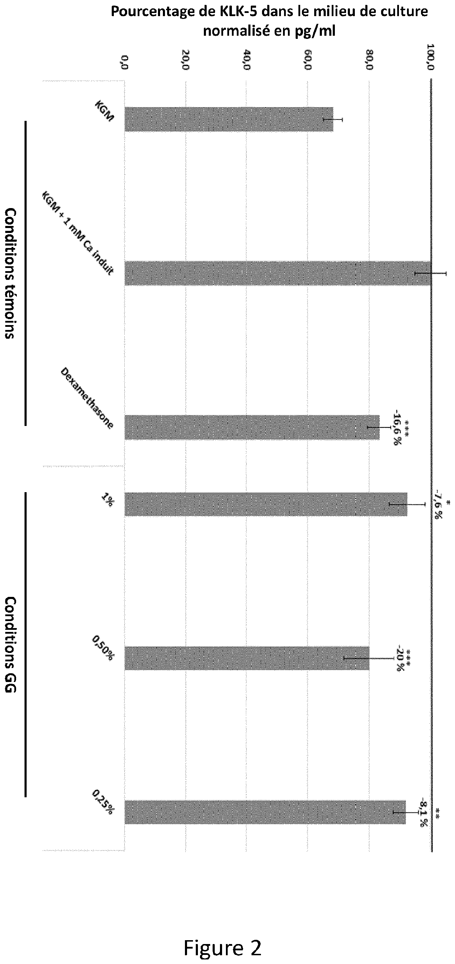

La figure 2 montre l'effet du polysaccharide glycogen (INCI ; dénommé ci-après gGG) sur l'inhibition de la synthèse de KLK-5 sécrétée par les kératinocytes humains normaux (KHN) après 48 heures d'incubation. Le témoin positif choisi est la dexaméthasone à 1µM. Les résultats sont exprimés sous forme de pourcentage d'expression normalisée par rapport à la viabilité cellulaire. Ils représentent les données obtenues sur 1 expérience réalisée en triplicate.

### Exemples de réalisation

### Exemple 1 : Composition cosmétique au sens de l'invention - émulsion H/E - crème fluide

Une composition selon l'invention est décrite dans le tableau 1.

| **Composé (désignation INCI)** | **Poids (en %)** |
|---|---|
| Water | 77,165 |
| Hydrogenated polydecene | 10,00 |
| Glycerin | 5,00 |
| Cetearyl alcohol | 2,40 |
| Phenoxyethanol | 1,015 |
| PEG-100 stéarate | 1,00 |
| Glyceryl stéarate | 1,00 |
| 1,2-hexanediol | 1,00 |
| Cetearyl glucoside | 0,60 |
| **Maltooligosyl glucoside** | 0,235 |
| Disodium EDTA | 0,20 |
| caprylyl glycol | 0,15 |
| **Hydrogenated starch hydrolysate** | 0,135 |
| Carbomer | 0,10 |

### Exemple 2 : Composition cosmétique (non revendiquée) - émulsion H/E - baume riche

Une composition est décrite dans le tableau 2

| **Composé (désignation INCI)** | **Poids (en %)** |
|---|---|
| Water | 64,51 |
| Caprylic/capric triglycéride | 9,00 |
| Glycerin | 8,00 |
| Olea europaea (olive) fruit oil | 7,00 |
| Mangifero indica (mango) seed butter | 3,00 |
| Sucrose stéarate | 2,50 |
| Sucrose distearate | 2,50 |
| C10-C18 triglycérides | 2,00 |
| **Glycogen** | 0,30 |

| | |
|---|---|
| Xanthan gum | 0,30 |
| Potassium sorbate | 0,20 |
| Sodium benzoate | 0,20 |
| Sodium citrate | 0,20 |
| Lactic acid | 0,19 |
| Fragrance | 0,1 |

### Exemple 3 : Actions des actifs sur la synthèse cellulaire de KLK-5 dans un environnement mimant la rosacée

### 1- But de l'étude

Le but de l'étude est de mettre en évidence l'effet d'actifs sur l'inhibition de la synthèse de la protéine KLK5 par des kératinocytes humains normaux (KHN) dans un environnement mimant la rosacée.

### 2- Matériels et méthodes

### 2.1- Culture cellulaire

Des KHN provenant de la société LONZA (France) ont été utilisés dans cette étude.

Les conditions de culture sont les suivantes :
- Type cellulaire : KHN donneur PP12
- Milieu de culture : KBM + compléments Lonza = KGM
- Densité d'ensemencement : 15 000 cellules/puits dans des plaques 96 puits avec 100 µl de milieu.

Les conditions d'induction de la synthèse de KLK-5 sont les suivantes :
- Présence de 1mM de Calcium
- Mix d'inducteur :(cf. Tableau 1) IL-17 à 10ng/ml + Vit D à 10-7 mol/L
- Temps de traitement : 48 heures
- Témoin positif d'inhibition de KLK5 induit : Dexaméthasone à 1µM

Les inducteurs de KLK-5 suivants listés dans le tableau 3 ont été utilisés dans l'étude :

| **Inducteur** | **Fournisseur** | **Concentration testée** | **Solution mère** |
|---|---|---|---|
| IL-17 | R&D systems | 10 ng/ml | 50 µg/ml |
| Vit D | Sigma | 10-⁷ M | 0,418 g/L |

### 2.2- Dosage de KLK-5 dans les surnageants de culture par ELISA

La quantification de KLK-5 dans les surnageants est réalisée grâce à un test ELISA, Enzyme Linked Immunosorbent Assay (Ref : DY8459-05, R&D systems, France) avec les réactifs recommandés par le fabricant (DuoSet Ancillary Reagent Kit 2, Ref : DY008, R&D systems, France) et utilisés selon les instructions du fabricant.

La Densité Optique (DO) est lue au spectrophotomètre à 450 nm. La concentration de KLK-5 est calculée à partir de la gamme étalon (15.6 à 1000 pg/ml) établie avec la protéine humaine recombinante de KLK-5.

Une dilution des surnageants au 1/50éme est obligatoire afin que les échantillons ne soient pas hors gammes. Les résultats sont exprimés en pg/ml de KLK-5 par puits. Sur le tapis cellulaire un test de viabilité au MTT ((3-4,5-dimethylthiazol-2-yl)-2,5-diphenyl tetrazolium bromide) est réalisé. Les dosages de KLK-5 sont exploités seulement si la viabilité cellulaire est supérieure à 80%, et sont rapportés à cette valeur de viabilité cellulaire.

### 2.3- Test de viabilité cellulaire au MTT

Le MTT est un colorant soluble jaune qui est métabolisé par les enzymes mitochondriales (succinate deshydrogenase) en un composé bleu foncé : le formazan. Les cristaux de formazan sont ensuite solubilisés dans du DMSO puis la densité optique est mesurée spectrophotométriquement à 540 nm. Cette méthode a été établie par Mosmann (1983), puis le protocole a ensuite été modifié par Denizot *et al.* (1986).

### 2.4- Protocole expérimental

Les KHN sont ensemencés dans du milieu KGM et incubés pendant 24h à 37°C en présence de 5% CO₂ avant le traitement avec les composés à l'étude dans du KGM et 1 mM de CaCl₂. Après 1 h d'incubation avec les actifs, l'induction de KLK-5 est effectuée avec l'association IL-17 à 10ng/ml + Vit D à 10-7 mol/L. Le KHN sont incubés pendant 48h à 37°C en présence de 5% CO₂; par la suite, les surnageants sont récupérés et les dosage ELISA de KLK-5 ainsi que le test de viabilité cellulaire au MTT sont effectués.

### 2.5- Actifs à l'étude

Les polysaccharides suivants listés dans le tableau 4 ont été testés :

| **Actifs (abréviation)** | **Nom INCI** |
|---|---|
| MG-60 (MOG+HHA) | Maltooligosyl glucoside & hydrogenated starch hydrolysate |
| Bioglycogen (GG) | glycogen |

Les actifs sélectionnés sont hydrosolubles.

La quantité de solvant dans les actifs a été ajustée à 0,1% ETOH : En effet il y a présence de 0,1% ETOH dans l'inducteur Vit D. Les résultats des actifs sont rapportés par rapport aux témoin solvant.

### 2.6- Analyses statistiques

Les données ont été collectées à partir d'expériences indépendantes réalisées en triplicat. Les analyses quantitatives sont exprimées sous forme de moyenne ± l'écart type. La significativité statistique est déterminée par un test de Student. Les différences sont considérées comme statistiquement significative à partir de p<0.05.
(NS : p>0.05 ; * : p≤0.05 ; ** : p≤0.01 ; *** : p≤0.001).

### 3- Résultats et conclusion

Les résultats sont représentés dans les figures 1 et 2 sous forme de graphique.

Conformément à ce qui était attendu pour la condition témoin, la dexaméthasone à 1µM inhibe la synthèse de KLK-5 de 16,6 %.

L'association de polysaccharides MOG+HHA (maltooligosyl glucoside & hydrogenated starch hydrolysate ; INCI) diminue la synthèse de KLK-5 de façon significative de 21,5%, 27,3% et 26,7% respectivement aux doses de 1%, 0,5% et 0,25% (figure 1)

Le polysaccharide GG (glycogen ; INCI) diminue la synthèse de KLK-5 de façon significative de 7,6%, 20% et 8,1% respectivement aux doses de 1%, 0,5% et 0,25%.

Cette étude démontre clairement que la combinaison des polysaccharides maltooligosyl glucoside & hydrogenated starch hydrolysate (INCI ; MOG + HHA ou encore le polysaccharide glycogen (INCI ; GG) diminuent de façon significative la synthèse de KLK-5 par les kératinocytes humains normaux cultivés dans un environnement mimant la rosacée.

## Revendications

1. Composition comprenant, en tant que principe actif dermatologique, au moins un polysaccharide consistant en :
- un hétéropolymère de glucose et tréhalose correspondant à la désignation INCI maltooligosyl glucoside ; et
- un homopolymère hydrogéné de glucose ramifié comprenant des liaisons α (1-4) et α (1-6) correspondant à la désignation INCI hydrogenated starch hydrolysate,
pour utilisation pour le traitement de la rosacée et/ou de la télangiectasie.

2. Composition pour son utilisation selon la revendication 1, **caractérisée en ce que** l'hétéropolymère de glucose et tréhalose représente entre 0,001% et 2% en poids de la composition, avantageusement entre 0,01% à 0,5%.

3. Composition pour son utilisation selon l'une des revendications précédentes, **caractérisée en ce que** l'homopolymère hydrogéné de glucose ramifié comprenant des liaisons α (1-4) et α (1-6) représente entre 0,001% et 2% en poids de la composition, avantageusement entre 0,01% et 0,5%.

4. Composition pour son utilisation selon l'une des revendications précédentes, **caractérisée en ce qu'**il s'agit d'une composition cosmétique.

## Patentansprüche

1. Zusammensetzung, die als dermatologischen Wirkstoff mindestens ein Polysaccharid enthält, bestehend aus:
- einem Heteropolymer aus Glucose und Trehalose entsprechend der INCI-Bezeichnung Maltooligosylglucosid; und
- einem hydrierten Homopolymer aus verzweigter Glucose, das aus a (1-4)- und a (1-6)-Bindungen besteht, entsprechend der INCI-Bezeichnung "hydrogenated starch hydrolysate", zur Verwendung bei der Behandlung von Rosazea und/oder Teleangiektasie.

2. Zusammensetzung für ihre Verwendung nach Anspruch 1, **dadurch gekennzeichnet, dass** das Heteropolymer aus Glucose und Trehalose zwischen 0,001 % und 2 % Gew.-% der Zusammensetzung ausmacht, vorzugsweise zwischen 0,01 % und 0,5 %.

3. Zusammensetzung für ihre Verwendung nach einem der vorhergehenden Ansprüche, **dadurch gekennzeichnet, dass** das hydrierte Homopolymer aus verzweigter Glucose, das aus a (1-4)- und a (1-6)-Bindungen besteht, zwischen 0,001 und 2 Gew.-% der Zusammensetzung, vorzugsweise zwischen 0,01 und 0,5 %, ausmacht.

4. Zusammensetzung für ihre Verwendung nach einem der vorhergehenden Ansprüche, **dadurch gekennzeichnet, dass** es sich um eine kosmetische Zusammensetzung handelt.

## Claims

1. Composition comprising, as dermatological active principle, at least one polysaccharide consisting of :
- a heteropolymer of glucose and trehalose corresponding to the INCI designation maltooligosyl glucoside; and
- a hydrogenated homopolymer of branched glucose comprising α (1-4) and α (1-6) bonds corresponding to the INCI designation hydrogenated starch hydrolysate,
for use for the treatment of rosacea and/or telangiectasia.

2. Composition for its use according to claim 1, **characterized in that** the heteropolymer of glucose and trehalose represents between 0.001% and 2% by weight of the composition, advantageously between 0.01% to 0.5%.

3. Composition for its use according to one of the preceding claims, **characterized in that** the hydrogenated homopolymer of branched glucose comprising α (1-4) and α (1-6) bonds represents between 0.001 % and 2% by weight of the composition, advantageously between 0.01% and 0.5%.

4. Composition for its use according to one of the preceding claims, **characterized in that** it is a cosmetic composition.
